# EUROPÄISCHE PATENTSCHRIFT

(11) **EP 3 127 664 B1**
(45) Veröffentlichungstag und Bekanntmachung des Hinweises auf die Patenterteilung: **09.10.2019**
(21) Anmeldenummer: 15180164.4
(22) Anmeldetag: 07.08.2015
(51) Int. Cl.: B25J 21/02

(54) **MASCHINE ZUM ASEPTISCHEN ABFÜLLEN VON FLÜSSIGKEITEN, VERFAHREN ZUM ÖFFNEN UND VERFAHREN ZUM SCHLIESSEN EINES ÖFFNUNGSFENSTERS IN EINER MASCHINE ZUM ASEPTISCHEN ABFÜLLEN VON FLÜSSIGKEITEN**
MACHINE FOR ASEPTIC FILLING OF LIQUIDS, METHOD FOR OPENING AND A METHOD OF CLOSING AN OPENING WINDOW IN A MACHINE FOR ASEPTIC FILLING OF LIQUIDS
MACHINE DE REMPLISSAGE ASEPTIQUE DE LIQUIDES, PROCEDE D'OUVERTURE ET PROCEDE DE FERMETURE D'UNE FENETRE D'OUVERTURE DANS UNE MACHINE DE REMPLISSAGE ASEPTIQUE DE LIQUIDES

(43) Veröffentlichungstag der Anmeldung: 08.02.2017
(73) Patentinhaber: INDAG Pouch Partners GmbH, 69214 Eppelheim (DE)
(72) Erfinder: Harth, Rolf, 69151 Neckargemünd (DE); Lechert, Frank, 69469 Weinheim (DE)
(74) Vertreter: Grünecker Patent- und Rechtsanwälte PartG mbB

(56) Entgegenhaltungen:
- EP-A1- 2 415 532
- CN-A- 102 090 979
- CN-U- 203 956 931
- CN-U- 204 319 339
- DE-A1-102007 030 789
- JP-A- 2006 349 139
- US-A1- 2005 225 936

## Beschreibung

Die Erfindung betrifft eine Maschine zum aseptischen Abfüllen von Flüssigkeiten gemäß dem Oberbegriff des Anspruchs 1, ein Verfahren zum Öffnen eines Öffnungsfensters in einer Maschine zum aseptischen Abfüllen von Flüssigkeiten nach Anspruch 7 und ein Verfahren zum Schließen eines Öffnungsfensters in einer Maschine zum aseptischen Abfüllen von Flüssigkeiten nach Anspruch 8.

### Stand der Technik

Es ist bekannt, bei Maschinen zum aseptischen Abfüllen von Flüssigkeiten Öffnungsfenster vorzusehen, die einen Zugriff in den sterilen Raum mittels Handschuhen ermöglichen, die ausgehend von einer Begrenzungswand in den sterilen Raum hineinreichen. Im Allgemeinen werden die Öffnungsfenster mittels Hebeln verklemmt, und die Position des Öffnungsfensters wird über einen Sicherheitsschalter abgefragt. Bei bekannten Systemen besteht daher die Möglichkeit, dass ein Öffnungsfenster mechanisch entriegelt wird, obwohl der Kontakt noch die Position "Zu" meldet. Somit kann z.B. Wasserstoffperoxid (H₂O₂), das als Desinfektions- und/oder Sterilisationsmittel vorgesehen ist, beim Öffnen des Fensters austreten, und die Aseptikmaschine verliert ihren Steril- bzw. Aseptikstatus, ohne dass die Steuerung diese Information erhält. Somit besteht die Gefahr, dass unerwartet eine Sekundärverkeimung des Produkts auftritt. Auch könnten die Bediener der Aseptikmaschine durch das austretende H₂O₂ gefährdet werden.

Die WO 2014/072229 A1 offenbart eine Inspektionsvorrichtung in Art eines Öffnungsfensters in einer Aseptikmaschine mit Handschuhen, die ins Innere des sterilen Raums ausgerichtet sind, wobei die Handschuhe an Innenwänden des sterilen Raums mittels Magnethalterung befestigbar sind. Zudem umfasst die Inspektionsvorrichtung eine Türe, die zwischen einer offenen und einer geschlossenen Position bewegbar ist und mittels der die Eingangsöffnungen in die Handschuhe verschlossen werden können.

Die CN 102090979 A offenbart eine aseptische Maschine, die zum aseptischen Abdichten zwischen dem Flügelrahmen und dem Fensterrahmen eine aufblasbare Dichtung umfasst.

### Aufgabe

Die Aufgabe der Erfindung besteht darin, Schwierigkeiten, die sich durch ein mechanisches Öffnen bzw. Schließen eines Öffnungsfensters ergeben, die eine Sekundärverkeimung des Produkts ermöglichen und eine Gefährdung der Bediener darstellen, zu vermeiden.

### Lösung

Die Aufgabe wird gelöst durch die Maschine nach Anspruch 1, das Verfahren nach Anspruch 7 und das Verfahren nach Anspruch 8. Bevorzugte Ausführungsformen und Weiterbildungen sind in den Unteransprüchen offenbart.

Erfindungsgemäß ist vorgesehen, dass die Maschine zum aseptischen Abfüllen von Flüssigkeiten einen Innenraum und eine Zugangsöffnung in einer Wandung der Maschine umfasst, die einen Zugang zu dem Innenraum ermöglicht. Die Zugangsvorrichtung umfasst ein Öffnungsfenster mit einem beweglichen Flügelrahmen und einem in der Wandung fest angeordneten Fensterrahmen, wobei zum aseptischen Abdichten zwischen dem Flügelrahmen und dem Fensterrahmen eine aufblasbare Dichtung vorgesehen ist..

Der Vorteil der aufblasbaren Dichtung besteht darin, dass eine aseptische Abdichtung erreicht werden kann, wobei das Öffnungsfenster aber trotzdem leicht öffenbar ist, da nach Ablassen der Luft aus der Dichtung diese schrumpft und somit die Kontaktfläche zu dem Fensterahmen deutlich verkleinert.

Als Flügelrahmen wird hierbei derjenige Teil des Öffnungsfensters bezeichnet der bewegbar, d.h. im speziellen zum Öffnen und Schließen vorgesehen ist, um so den Zugang zum Innenraum der Maschine zu ermöglichen.

Als Fensterrahmen wird hierbei derjenige Teil des Öffnungsfensters bezeichnet, der nicht bewegbar ausgebildet ist. Die Wandung trennt einen Außenraum von dem Innenraum der Maschine ab.

Eine aufblasbare Dichtung besteht im Allgemeinen aus einem geschlossenen, hohlen Ring aus einem elastischen Material, in den Luft und/oder ein sonstiges Gas eingebracht werden kann. Wenn im Folgenden von Luft die Rede ist, ist immer auch Gas oder ein Gas-LuftGemisch gemeint. Die Dichtung, d.h. der Ring, dehnt sich bei der Füllung mit Luft aus und passt sich der Form der anliegenden Oberflächen an. Durch eine ausreichend große Füllung mit Luft wird genügend Druck aufgebaut und so ein aseptisches Abdichten zwischen dem Flügelrahmen und dem Fensterrahmen erreicht. Wird die Luft wieder aus der aufblasbaren Dichtung abgelassen, so schrumpft die aufblasbare Dichtung, d.h. der Ring, und der Zustand der aseptischen Abdichtung ist aufgehoben. Der Ausdruck "aseptisch" bedeutet hier und im Folgenden steril bzw. keimfrei, d.h. frei von fortpflanzungsfähigen Mikroorganismen sowie deren Ruhestadien oder Dauerformen, wie z.B. Sporen. Ein aseptisches Abdichten eines Raums bedeutet, dass durch das Abdichten ein aseptischer Zustand des Raums erreicht wird.

Das Öffnungsfenster kann derart dimensioniert sein, dass es den Zugang zu zwei Handschuhöffnungen gewährleistet, in die eine Bedienperson mit ihren Händen hineingreifen kann, um so Arbeiten im Inneren der Maschine vornehmen zu können. Das Öffnungsfenster kann auch größer oder kleiner ausgeführt sein, je nach Art des gewünschten Zugangs zu dem Innenraum der Aseptikmaschine.

Die Maschine kann in der lebensmittel- und/oder tierfutterverarbeitenden Industrie vorgesehen sein.

In dem Flügelrahmen kann eine Nut angeordnet sein, in welcher die aufblasbare Dichtung in einem nicht aufgeblasenen Zustand zumindest teilweise angeordnet ist. Durch diese Anordnung der aufblasbaren Dichtung in der Nut, kann bei geöffnetem Öffnungsfenster vermieden werden, dass die aufblasbare Dichtung beschädigt oder durch Kontakt mit den Händen eines Bedieners, beispielsweise durch Hautfett, verunreinigt wird. Zudem kann ein geschlossenes Öffnungsfenster, bei dem bereits die Luft aus der aufblasbaren Dichtung entfernt wurde, nach einem Entriegeln leichter geöffnet werden, da sich die geschrumpfte Dichtung in die Nut zurückziehen kann, so dass ein Kontakt des Gummimaterials mit dem Fensterrahmen vermieden wird. Dadurch wird keine zusätzliche Kraft zum Öffnen benötigt, um einen möglicherweise klebenden oder anhaftenden Kontakt zwischen dem Gummimaterial der Dichtung und dem Material des Fensterrahmens zu überwinden.

In einem geschlossenen Zustand des Öffnungsfensters ist außerdem ein Ausdehnungsbereich zwischen dem Fensterrahmen und dem Flügelrahmen vorgesehen, der derart ausgebildet ist, dass sein Querschnitt zum Innenraum der Maschine hin zunimmt. Vorzugsweise nimmt der Querschnitt kontinuierlich zu. Durch das Vorsehen dieses zusätzlichen Ausdehnungsbereichs für die aufblasbare Dichtung kann in gewissem Maße gesteuert werden, wie die aseptische Abdichtung zwischen dem Flügelrahmen und dem Fensterrahmen erfolgt. Durch die angegebene Ausbildung das Ausdehnungsbereichs, d.h. die Zunahme des Querschnitts, wird erreicht, dass bei einem geschlossenen Öffnungsfenster, wenn die aufblasbare Dichtung mit Luft gefüllt ist, der Dichtdruck zu dem Innenraum der Maschine hin größer ist.

Das Öffnungsfenster kann weiter ein mittels eines Steuersystems steuerbares Ventil für eine Luftversorgung der aufblasbaren Dichtung umfassen. Statt einer Luftversorgung kann auch eine Gasversorgung vorgesehen sein. Das Steuersystem des steuerbaren Ventils ermöglicht somit ein effizientes und genaues Versorgen der aufblasbaren Dichtung mit Luft, um die aufblasbare Dichtung mit Luft aufzublasen bzw. Luft aus der aufblasbaren Dichtung abzulassen. Das Öffnungsfenster kann weiter einen magnetischen Schalter mit einem Kontakt, der am oder im Flügelrahmen und ein Gegenstück des magnetischen Schalters, das an dem oder in dem Fensterrahmen angeordnet ist, umfassen. Der Kontakt des magnetischen Schalters kann dazu verwendet werden, zu verhindern, dass das Öffnungsfenster mechanisch entriegelt wird, obwohl der Kontakt noch die Position "zu" meldet.

Vorzugsweise sind der magnetische Schalter und der Kontakt in einem Griff vorgesehen, der am Flügelrahmen angeordnet ist.

Ein Verfahren zum Öffnen eines Öffnungsfensters in einer Maschine zum aseptischen Abfüllen, wie oben oder weiter unten beschrieben, umfasst mehrere Schritte. In einem Schritt erfolgt ein Stellen einer Anforderung mit einem ersten Signal zum Öffnen des Öffnungsfensters. Dazu kann angenommen werden, dass sich das Öffnungsfenster zuvor in einem geschlossenen Zustand befindet, der den Innenraum der Maschine aseptisch vom Außenraum der Maschine abschließt.

In einem nächsten Schritt erfolgt ein Übermitteln durch ein zweites Signal, dass der Aseptikraum sicher zu öffnen ist. Dementsprechend erfolgt in einem darauffolgenden Schritt ein Veranlassen von Ablassen der Luft aus der aufblasbaren Dichtung mit einem dritten Signal. Vorzugsweise erfolgt das Ablassen der Luft mittels des Ventils.

In einem nächsten Schritt erfolgt ein Übermitteln durch ein viertes Signal, dass sich keine Luft in der aufblasbaren Dichtung befindet und danach Entriegeln des Magnets durch ein fünftes Signal. Somit kann das Öffnungsfenster dann geöffnet werden.

Durch dieses Verfahren zum Öffnen des Öffnungsfensters kann vermieden werden, dass eine Maschine zum aseptischen Abfüllen von Flüssigkeiten unerwartet ihren Steril- bzw. Aseptikstatus beispielsweise während eines Füllvorgangs verliert und es dadurch zu einer Sekundärverkeimung des abgefüllten bzw. abzufüllenden Produkts, d.h. einer Flüssigkeit, kommt. Es ist einem Bediener nicht möglich, das Öffnungsfenster zu öffnen, bis alle Verfahrensschritte hin zur Entriegelung des Magnets durchlaufen wurden.

Bei diesem Verfahren zum Öffnen eines Öffnungsfensters handelt es sich vorzugsweise um ein computer-implementiertes Verfahren. Dazu können auf einem computerlesbaren Speichermedium Instruktionen gespeichert sein, die durch einen Prozessor eines Computers ausführbar sind und den Computer dazu veranlassen, die Verfahrensschritte auszuführen.

Ein Verfahren zum Schließen eines Öffnungsfensters in einer Maschine zum aseptischen Abfüllen, wie oben oder weiter unten beschrieben, umfasst mehrere Schritte. In einem ersten Schritt erfolgt ein Stellen einer Anforderung mit einem ersten Signal zum Schließen des Öffnungsfensters. Dazu wird angenommen, dass sich das Öffnungsfenster zuvor in einem offenen Zustand befindet. Als offener Zustand wird hierbei angesehen, dass der Flügelrahmen in den Fensterrahmen eingebracht ist, das Öffnungsfenster aber nicht verriegelt ist.

In einem nächsten Schritt erfolgt ein Übermitteln durch ein zweites Signal, dass der Magnet verriegeln soll. Dadurch verriegelt der Magnet und anschließend erfolgt ein Übermitteln durch ein drittes Signal, dass der Magnet verriegelt ist. Danach erfolgt ein Übermitteln des dritten Signals an das Ventil und Einbringen von Druckluft in die aufblasbare Dichtung.

Dann erfolgt ein Übermitteln durch ein viertes Signal, dass Druckluft in der aufblasbaren Dichtung vorhanden ist, und im nächsten Schritt erfolgt ein Übermitteln durch ein fünftes Signal, dass der Aseptikraum, d.h. der Innenraum der Maschine zum aseptischen Abfüllen von Flüssigkeiten, sicher verschlossen ist.

Dieses Verfahren zum Schließen des Öffnungsfensters bietet den Vorteil, dass das Öffnungsfenster mittels des Magneten verriegelt wird, bevor Druckluft in die aufblasbare Dichtung eingebracht wird. Durch das Verriegeln des Magnets befinden sich Flügelrahmen und Fensterrahmen somit immer in einer definierten Position zueinander. Dadurch kann vermieden werden, dass ein Einbringen von Druckluft in die aufblasbare Dichtung erfolgt, wenn der Flügelrahmen zwar in den Fensterrahmen eingebracht ist, sich aber nicht einer solchen Position befindet, die dann ein aseptisches Abdichten ermöglichen würde.

Bei diesem Verfahren zum Schließen eines Öffnungsfensters handelt es sich vorzugsweise um ein computer-implementiertes Verfahren. Dazu können auf einem computerlesbaren Speichermedium Instruktionen gespeichert sein, die durch einen Prozessor eines Computers ausführbar sind und den Computer dazu veranlassen, die Verfahrensschritte auszuführen.

Die beigefügten Figuren stellen beispielhaft zum besseren Verständnis und zur Veranschaulichung Aspekte der Erfindung dar. Es zeigt:
Figur 1 eine Frontansicht eines Öffnungsfensters,
Figur 2 eine Seitenansicht des Öffnungsfensters,
Figur 3 eine Schnittansicht entlang der Achse A-A aus Figur 1,
Figur 4 eine Ausschnittvergrößerung des Ausschnitts B der Figur 3,
Figur 5 eine Schnittansicht entlang der Achse C-C aus Figur 1,
Figur 6 eine Ausschnittvergrößerung des Ausschnitts D der Figur 5,
Figur 7 ein Flussdiagramm eines Verfahrens zum Öffnen eines Öffnungsfensters und
Figur 8 ein Flussdiagramm eines Verfahrens zum Schließen des Öffnungsfensters.

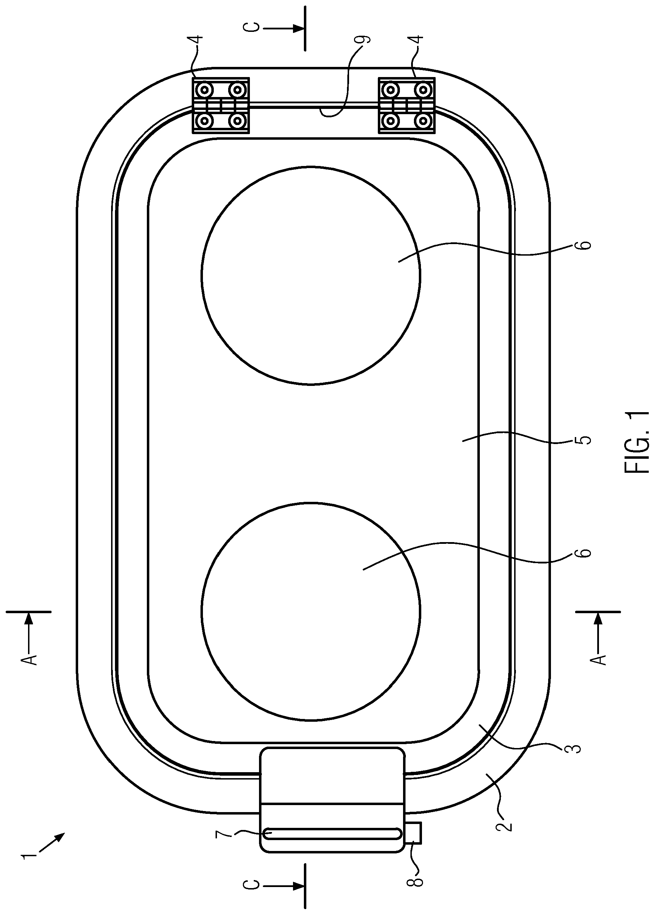

Figur 1 zeigt eine Frontansicht eines Öffnungsfensters 1, das in einer Maschine zum aseptischen Abfüllen verwendet werden kann. Das dargestellte Öffnungsfenster 1 umfasst einen Fensterrahmen 2 und einen Flügelrahmen 3, wobei diese beiden Rahmen mittels zweier Scharniere 4 verbunden sind, so dass der Flügelrahmen 3 relativ zu dem Fensterrahmen 2 bewegt werden kann, um das Öffnungsfenster 1 zu öffnen oder zu schließen. Der Flügelrahmen 3 umfasst zudem einen magnetischen Schalter 8, der zu einer Verriegelung des Öffnungsfensters 1 im geschlossenen Zustand dient. Das gezeigte Öffnungsfenster 1 ist für einen Zugang zum Innenraum der Maschine vorgesehen, wobei der Fensterrahmen 2 an einer nicht gezeigten Wandung, welche die Maschine umgibt, angeordnet ist. Das Fenster 5 des Öffnungsfensters 1, das sich im Flügelrahmen 3 befindet, ist hier durchsichtig ausgeführt, sodass der Blick auf zwei Zugangsöffnungen 6 für Handschuhe sichtbar ist. Der magnetische Schalter 8 ist in bzw. an einem Griff 7 des Flügelrahmens 3 angeordnet, so dass einem Bediener durch den Griff 7 ein leichtes Öffnen bzw. Schließen des Öffnungsfensters 1 ermöglicht wird. Um für eine Verriegelung des Öffnungsfensters 1 zu sorgen, ist an dem Fensterrahmen 2 ein nicht dargestelltes Gegenstück des magnetischen Schalters angeordnet. Mittels des Ventils 9 kann in die nicht dargestellte aufblasbare Dichtung, die zwischen dem Fensterrahmen 2 und dem Flügelrahmen 3 angeordnet ist, Luft eingebracht bzw. Luft daraus entfernt werden.

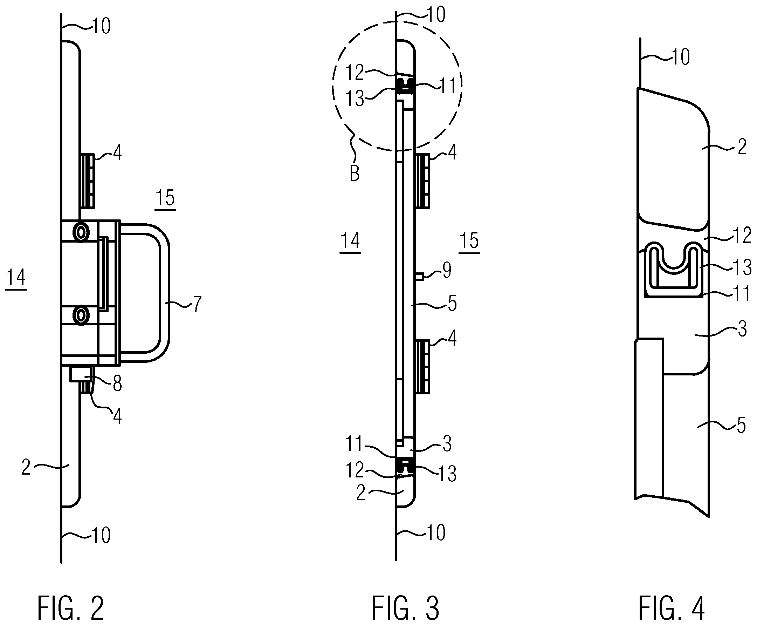

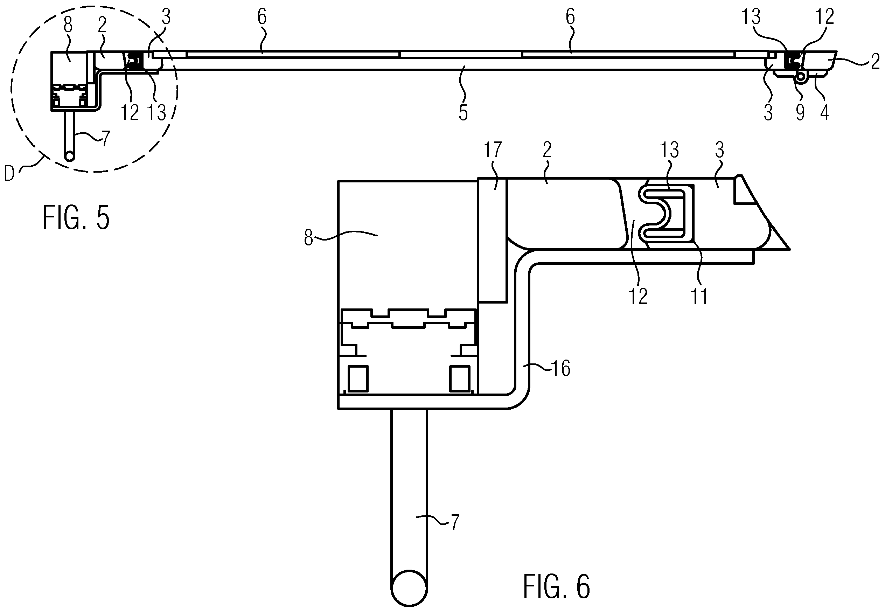

In der Figur 1 sind zudem die beiden Schnittachsen A-A und C-C dargestellt, wobei die entsprechenden Darstellungen des Öffnungsfensters 1 in den Figuren 3 bzw. 5 gezeigt werden.

Figur 2 zeigt eine Seitenansicht des Öffnungsfensters 1, das an einer Wandung 10, welche die Maschine umgibt, angeordnet ist. Hierbei sind der an dem Flügelrahmen 3 angeordnete Griff 7 mit dem Kontakt 8 und der Fensterrahmen 2 des Öffnungsfensters 1 zu sehen sowie die beiden Scharniere 4, die den Flügelrahmen 3 und den Fensterrahmen 2 verbinden. Durch die Wandung 10 wird der Innenraum 14 der Maschine vom umgebenden Außenraum 15 abgegrenzt.

Figur 3 zeigt eine Schnittansicht entlang der Achse A-A aus Figur 1. Der Fensterrahmen 2 sitzt hierbei auf dieser Wandung 10 auf bzw. ist daran befestigt und begrenzt einen Zugangsbereich des Öffnungsfensters 1, in dem sich auch die beiden Zugangsöffnungen 6 für die Handschuhe befinden. In der dargestellten geschlossenen Position des Öffnungsfensters 1 befindet sich in der Flügelrahmen 3 derart benachbart zu dem Fensterrahmen 2, so dass die aufblasbare Dichtung 13, die in einer Nut 11 des Flügelrahmens 3 angeordnet ist, sich in einen Ausdehnungsbereich 12, der zwischen dem Flügelrahmen 3 und dem Fensterrahmen 2 besteht, hinein ausdehnen kann, wenn sie mit Luft gefüllt wird. In Figur 3 ist die aufblasbare Dichtung 13 in einem ungefüllten Zustand gezeigt.

Der Ausdehnungsbereich 12 ist derart ausgebildet, dass sein Querschnitt bei geschlossenem Öffnungsfenster 1 zum Innenraum 14 der Maschine hin zunimmt. Der gekennzeichnete Bereich B wird in der Figur 4 näher erläutert.

Figur 4 zeigt eine Ausschnittvergrößerung des Ausschnitts B der Figur 3. Hier ist deutlich die Nut 11 für die aufblasbare Dichtung 13 in dem Flügelrahmen 3 zu sehen sowie die Form des Ausdehnungsbereichs 12 zwischen dem Flügelrahmen 3 und dem Fensterrahmen 2, der im Dreidimensionalen eine annähernd trapezförmige Form aufweist. Durch diese Form des Ausdehnungsbereichs 12 ergibt sich bei geschlossenem Öffnungsfenster 1 und mit Luft gefüllter aufblasbarer Dichtung 13 nach innen hin, d.h. zum Innenraum 14 der Aseptikmaschine, ein größerer Druck, so dass dadurch der Flügelrahmen 3 in den Fensterrahmen 2 gezogen wird, was eine Ausbildung der aseptischen Abdichtung verbessert.

Figur 5 zeigt eine Schnittansicht entlang der Achse C-C aus Figur 1. Hier wird noch einmal die relative Anordnung des Flügelrahmens 3 in Bezug zu dem Fensterahmen 2 deutlich. Zudem ist das Ventil 9 erkennbar, dass für eine Luftversorgung der aufblasbaren Dichtung 13 vorgesehen ist. Der gekennzeichnete Bereich D wird in Figur 6 näher erläutert.

Figur 6 zeigt eine Ausschnittvergrößerung des Ausschnitts D der Figur 5. In Figur 6 ist der Bereich des Öffnungsfensters 1, der den Griff 7 mit dem magnetischen Schalter 8 umfasst, gezeigt. Der Griff 7 ist hierbei mittels einer Verbindungsvorrichtung 16 mit dem Flügelrahmen 3des Öffnungsfensters 1 verbunden. Der magnetische Schalter 8, der in dem Griff 7 bzw. daran angeordnet ist, umfasst einen Kontakt 17, der in einem geschlossenen Zustand des Öffnungsfensters 1 freigegeben wird bzw. in einem verriegelten Zustand des Öffnungsfensters 1 belegt ist. Somit kann der Kontakt 17 des magnetischen Schalters 8 verwendet werden, um ein sicheres Verschließen und Verschlossenbleiben des Öffnungsfensters 1 zu gewährleisten. Der magnetische Schalter 8 verhindert ein mechanisches Öffnen des Öffnungsfensters 1, solange die aseptische Abdichtung zwischen dem Flügelrahmen 3 und dem Fensterrahmen 2 besteht.

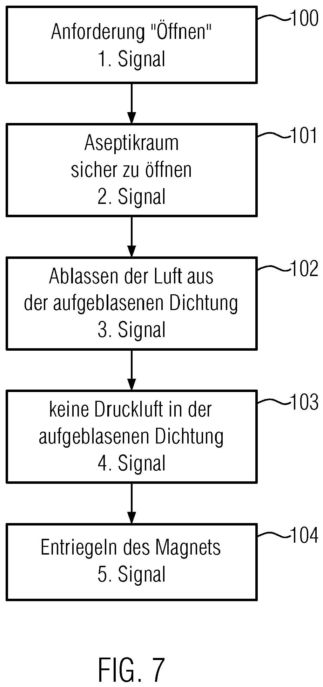

Figur 7 zeigt ein Flussdiagramm eines Verfahrens zum Öffnen eines Öffnungsfensters einer Maschine zum aseptischen Abfüllen wie oben oder weiter unten beschrieben. Dabei handelt es sich vorzugsweise um ein computer-implementiertes Verfahren. Dazu können auf einem computerlesbaren Speichermedium Instruktionen gespeichert sein, die durch einen Prozessor eines Computers ausführbar sind und den Computer dazu veranlassen, die Verfahrensschritte auszuführen.

Im Schritt 100 wird mit einem ersten Signal eine Anforderung zum Öffnen des Öffnungsfensters gestellt. Dazu wird angenommen, dass sich das Öffnungsfenster zuvor in einem geschlossenen Zustand befindet, der den Innenraum der Maschine aseptisch vom Außenraum der Maschine abschließt.

Im Schritt 101 wird durch ein zweites Signal übermittelt, dass der Aseptikraum sicher zu öffnen ist. Dementsprechend wird im Schritt 102 mit einem dritten Signal ein Ablassen der Luft aus der aufblasbaren Dichtung veranlasst. Vorzugsweise erfolgt das Ablassen der Luft mittels des Ventils.

Im Schritt 103 wird durch ein viertes Signal übermittelt, dass sich keine Druckluft in der aufblasbaren Dichtung befindet. Danach erfolgt im Schritt 104 durch ein fünftes Signal ein Entriegeln des Magnets, so dass das Öffnungsfenster geöffnet werden kann.

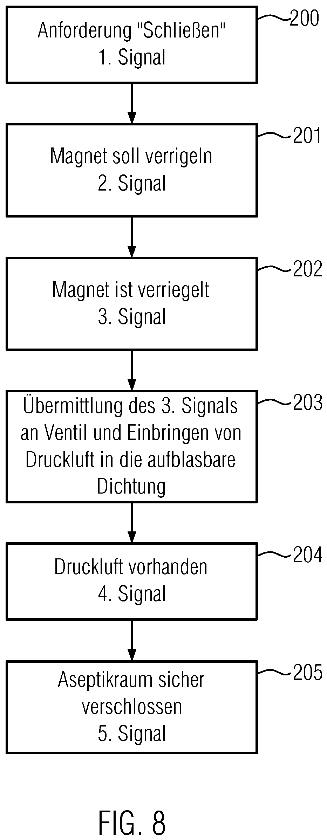

Figur 8 zeigt ein Flussdiagramm eines Verfahrens zum Schließen des Öffnungsfensters einer Maschine zum aseptischen Abfüllen wie oben oder weiter unten beschrieben. Dabei handelt es sich vorzugsweise um ein computer-implementiertes Verfahren. Dazu können auf einem computerlesbaren Speichermedium Instruktionen gespeichert sein, die durch einen Prozessor eines Computers ausführbar sind und den Computer dazu veranlassen, die Verfahrensschritte auszuführen.

Im Schritt 200 wird mit einem ersten Signal eine Anforderung zum Schließen des Öffnungsfensters gestellt. Dazu wird angenommen, dass sich das Öffnungsfenster zuvor in einem offenen Zustand befindet. Als offener Zustand wird hierbei angesehen, dass der Flügelrahmen in den Fensterrahmen eingebracht ist, das Öffnungsfenster aber nicht verriegelt ist.

Im Schritt 201 wird durch ein zweites Signal übermittelt, dass der Magnet verriegeln soll. Dadurch verriegelt der Magnet und anschließend wird im Schritt 202 durch ein drittes Signal übermittelt, dass der Magnet verriegelt ist. Dann wird im Schritt 203 das dritte Signal an das Ventil übermittelt und Druckluft in die aufblasbare Dichtung eingebracht.

Im Schritt 204 wird durch ein viertes Signal übermittelt, dass Druckluft in der aufblasbaren Dichtung vorhanden ist, und im Schritt 205 wird durch ein fünftes Signal übermittelt, dass der Aseptikraum, d.h. der Innenraum der Maschine zum aseptischen Abfüllen von Flüssigkeiten, sicher verschlossen ist.

## Patentansprüche

1. Maschine zum aseptischen Abfüllen von Flüssigkeiten mit einem Innenraum (14) und mit einer Zugangsöffnung in einer Wandung (10) der Maschine, die einen Zugang zu dem Innenraum (14) ermöglicht, wobei die Zugangsvorrichtung ein Öffnungsfenster (1) mit einem beweglichen Flügelrahmen (3) und einem in der Wandung (10) fest angeordneten Fensterrahmen (2) umfasst, wobei zum aseptischen Abdichten zwischen dem Flügelrahmen (3) und dem Fensterrahmen (2) eine aufblasbare Dichtung (13) vorgesehen ist,
**dadurch gekennzeichnet, dass**
im geschlossenen Zustand des Öffnungsfensters (1) ein Ausdehnungsbereich (12) zwischen dem Flügelrahmen (3) und dem Fensterrahmen (2) für die aufblasbare Dichtung (13) vorgesehen ist, der derart ausgebildet ist, dass sein Querschnitt zum Innenraum (14) der Aseptikmaschine hin zunimmt.

2. Maschine nach Anspruch 1, bei der weiter eine Nut (11) in dem Flügelrahmen (3) angeordnet ist, in welcher die aufblasbare Dichtung (13) in einem nicht aufgeblasenen Zustand zumindest teilweise angeordnet ist.

3. Maschine nach einem der Ansprüche 1 bis 2, wobei das Öffnungsfenster (1) weiter ein mittels eines Steuersystems steuerbares Ventil (9) für eine Luftversorgung der aufblasbaren Dichtung (13) umfasst.

4. Maschine nach einem der Ansprüche 1 bis 3, die weiter einen magnetischen Schalter (8) mit einem Kontakt (17), der am Flügelrahmen (3) und ein Gegenstück des magnetischen Schalters, das am Fensterrahmen (2) angeordnet ist, umfasst.

5. Maschine nach Anspruch 4, wobei der magnetische Schalter (8) und der Kontakt (17) an oder in einem Griff (7) vorgesehen sind, der am Flügelrahmen (3) angeordnet ist.

6. Verfahren zum Öffnen eines Öffnungsfensters in einer Maschine zum aseptischen Abfüllen von Flüssigkeiten nach Anspruch 4 oder 5 mit den Schritten:
- Stellen (100) einer Anforderung mit einem ersten Signal zum Öffnen des Öffnungsfensters,
- Übermitteln (101) durch ein zweites Signal, dass der Aseptikraum sicher zu öffnen ist,
- Veranlassen (102) von Ablassen der Luft aus der aufblasbaren Dichtung mit einem dritten Signal,
- Übermitteln (103) durch ein viertes Signal, dass sich keine Luft in der aufblasbaren Dichtung befindet und
- danach Entriegeln (104) des Magnets durch ein fünftes Signal.

7. Verfahren zum Schließen eines Öffnungsfensters in einer Maschine zum aseptischen Abfüllen von Flüssigkeiten nach Anspruch 4 oder 5 mit den Schritten:
- Stellen (200) einer Anforderung mit einem ersten Signal zum Schließen des Öffnungsfensters,
- Übermitteln (201) durch ein zweites Signal, dass der Magnet verriegeln soll,
- Übermitteln (202) durch ein drittes Signal, dass der Magnet verriegelt ist,
- Übermitteln (203) des dritten Signals an das Ventil und Einbringen (203) von Druckluft in die aufblasbare Dichtung,
- Übermitteln (204) durch ein viertes Signal, dass Druckluft in der aufblasbaren Dichtung vorhanden ist und
- Übermitteln (205) durch ein fünftes Signal, dass der Aseptikraum, d.h. der Innenraum der Maschine zum aseptischen Abfüllen von Flüssigkeiten, sicher verschlossen ist.

## Claims

1. A machine for aseptic filling of liquids comprising an interior chamber (14) and an access opening in a wall (10) of the machine, the access opening enabling access to the interior chamber (14), wherein the access device comprises an opening window (1) including a movable sash (3) and a window frame (2) permanently attached to the wall (10), wherein for the aseptic sealing an inflatable seal (13) is provided between the sash (3) and the window frame (2),
**characterised in that**
in the closed state of the opening window (1), an expansion area (12) for the inflatable seal (13) is provided between the sash (3) and the window frame (2), the expansion area being configured such that its cross section increases towards the interior chamber (14) of the aseptic machine.

2. The machine of claim 1, further comprising a groove (11) positioned in the sash (3) in which the inflatable seal (13) in a non-inflated state is at least partially arrangeable.

3. The machine of claims 1 to 2, wherein the opening window (1) further comprises a valve (9) that is controllable by a control system and is used for air supply of the inflatable seal (13).

4. The machine of any of claims 1 to 3, further comprising a magnetic switch (8) including a contact (17) and attached to the sash (3), and a counterpart of the magnetic switch attached to the window frame (2).

5. The machine of claim 4, wherein the magnetic switch (8) and the contact (17) are provided on or in a handle (7) attached to the sash (3).

6. A method for opening an opening window in a machine for the aseptic filling of liquids of claim 4 or 5, the method comprising the steps:
- requesting (100), by a first signal, the opening of the opening window,
- communicating (101) a second signal indicating that the aseptic space is to be opened safely,
- causing (102) a removal of air from the inflatable seal via a third signal,
- communicating (103) a fourth signal indicating that no air is present in the inflatable seal and
- thereafter, unlocking (104) the magnet by a fifth signal.

7. A method for closing an opening window in a machine for the aseptic filling of liquids of claim 4 or 5, the method comprising the steps:
- requesting (200), by a first signal, the closing of the opening window,
- communicating (201) a second signal indicating that the magnet is to be locked,
- communicating (202) a third signal indicating that the magnet is locked,
- communicating (203) the third signal to the valve and introducing (203) pressurized air into the inflatable seal,
- communicating (204) a fourth signal indicating that pressurized air is present in the inflatable seal and
- communicating (205) a fifth signal indicating that the aseptic space, that is, the interior chamber of the machine for the aseptic filling of liquids, is safely locked.

## Revendications

1. Machine destinée au conditionnement par remplissage, aseptisé, de liquides, comportant une chambre intérieure (14) et une ouverture d'accès dans une paroi (10) de la machine, qui permet d'accéder à la chambre intérieure (14), machine
dans laquelle le dispositif d'accès comprend un fenêtre d'ouverture (1) avec un châssis de vantail (3) mobile et un châssis dormant de fenêtre (2) agencé de manière fixe dans la paroi (10), et
dans laquelle il est prévu, pour assurer une étanchéité aseptisée entre le châssis de vantail (3) et le châssis dormant de fenêtre (2), un joint d'étanchéité (13) gonflable,
**caractérisée en ce que** dans l'état fermé de la fenêtre d'ouverture (1), il est prévu une zone d'expansion (12) entre le châssis de vantail (3) et le châssis dormant de fenêtre (2), pour le joint d'étanchéité (13) gonflable, la zone d'expansion étant réalisée de manière telle, que sa section transversale augmente en direction de la chambre intérieure (14) de la machine aseptisée.

2. Machine selon la revendication 1, dans laquelle une rainure (11) est par ailleurs agencée dans le châssis de vantail (3), rainure dans laquelle est disposé, au moins en partie, le joint d'étanchéité (13) gonflable, dans un état non gonflé.

3. Machine selon l'une des revendications 1 à 2, dans laquelle la fenêtre d'ouverture (1) comprend, en outre, une valve (9) pouvant être commandée par un système de commande, pour une alimentation en air du joint d'étanchéité (13) gonflable.

4. Machine selon l'une des revendications 1 à 3, qui comprend, en outre, un commutateur magnétique (8) avec un contact (17) agencé sur le châssis de vantail (3), et une pièce conjuguée du commutateur magnétique, qui est agencée sur le châssis dormant de fenêtre (2).

5. Machine selon la revendication 4, dans laquelle le commutateur magnétique (8) et le contact (17) sont prévus sur ou dans une poignée (7), qui est agencée sur le châssis de vantail (3).

6. Procédé pour ouvrir une fenêtre d'ouverture dans une machine destinée au conditionnement par remplissage, aseptisé, de liquides, selon la revendication 4 ou la revendication 5, comprenant les étapes suivantes consistant à :
- formuler (100) une demande, à l'aide d'un premier signal, pour ouvrir la fenêtre d'ouverture,
- indiquer (101) par un deuxième signal, le fait que la chambre aseptisée peut être ouverte de manière sûre,
- provoquer (102) l'évacuation de l'air du joint d'étanchéité gonflable, à l'aide d'un troisième signal,
- indiquer (103) par un quatrième signal, qu'il n'y a plus d'air dans le joint d'étanchéité gonflable, et
- ensuite, déverrouiller (104) l'aimant par un cinquième signal.

7. Procédé pour fermer une fenêtre d'ouverture dans une machine destinée au conditionnement par remplissage, aseptisé, de liquides, selon la revendication 4 ou la revendication 5, comprenant les étapes suivantes consistant à :
- formuler (200) une demande, à l'aide d'un premier signal, pour fermer la fenêtre d'ouverture,
- indiquer (201) par un deuxième signal, que l'aimant doit verrouiller,
- indiquer (202) par un troisième signal, que l'aimant est verrouillé,
- transmettre (203) le troisième signal à la valve, et introduire (203) de l'air comprimé dans le joint d'étanchéité gonflable,
- indiquer (204) par un quatrième signal, la présence d'air comprimé dans le joint d'étanchéité gonflable, et
- indiquer (205) par un cinquième signal, le fait que la chambre aseptisée, c'est-à-dire la chambre intérieure de la machine destinée au conditionnement par remplissage, aseptisé, de liquides, est fermée de manière sûre.
